(19) Europäisches Patentamt
European Patent Office
Office européen des brevets

(11) EP 0 486 024 B1

## (12) EUROPEAN PATENT SPECIFICATION

(45) Date of publication and mention
of the grant of the patent:
**18.09.1996  Bulletin 1996/38**

(21) Application number: 91119453.8

(22) Date of filing: 14.11.1991

(51) Int Cl.6: **C12P 7/18**, C12N 1/20,
C12P 7/54, C12P 7/56,
C07C 51/47
// (C12N1/20, C12R1:225),
(C12P7/18, C12R1:225),
(C12P7/54, C12R1:225),
(C12P7/56, C12R1:225)

(54) **Lactobacillus SP.B001 and method of producing mannitol**

Laktobazillus sp.B001 und Verfahren zur Herstellung von Mannitol

Lactobacillus sp.B001 et méthode de productions de mannitol

(84) Designated Contracting States:
**CH ES FR GB IT LI NL**

(30) Priority: **15.11.1990  JP 307147/90**
**06.03.1991  JP 63770/91**

(43) Date of publication of application:
**20.05.1992  Bulletin 1992/21**

(60) Divisional application: 95111207.7

(73) Proprietors:
• **SUMITOMO HEAVY INDUSTRIES, LTD**
**Tokyo 100 (JP)**
• **KATOH KAGAKU KABUSHIKIGAISHA**
**Chita-gun, Aichi-ken (JP)**

(72) Inventors:
• **Itoh, Yoshikuni**
**Chita-gun, Aichi-ken (JP)**
• **Ogasawara, Kengi**
**Chita-gun, Aichi-ken (JP)**
• **Inaba,Hideki, Seimeiryo**
**Kanagawa-ken (JP)**
• **Sakamoto, Yoichiro, 67-6-1, Matoi**
**Kanagawa-ken (JP)**
• **Koga, Junko, Minamihara-apahto**
**Kanagawa-ken (JP)**

(74) Representative: **Kahler, Kurt, Dipl.-Ing.**
**Patentanwälte Kahler, Käck, Fiener & Sturm**
**P.O. Box 12 49**
**87712 Mindelheim (DE)**

(56) References cited:
CH-A- 388 282          GB-A- 3 261

• **PATENT ABSTRACTS OF JAPAN, vol. 12, no.
117 (C-487)[2964], 13 April 1988**
• **JOURNAL OF APPLIED BACTERIOLOGY, vol.
65, no. 2, August 1988; R.P. TRACEY et al., pp.
113-118**
• **ASSOCIATION OF OFFICIAL ANALYTICAL
CHEMISTS JOURNAL, vol. 67, no. 4, July/August
1984; R.F. McFEETERS et al., pp. 710-714**
• **BULLETIN DE LA SOCIETE CHIMIQUE DE
FRANCE, 1963, Paris (FR); A.J. COURTOISIER et
al., pp. 350-355**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

BACKGROUND OF THE INVENTION

The present invention relates to a new bacterial strain per se of lactic acid bacteria, and a method of producing mannitol using this strain. Still more particularly, the present invention relates to a new bacterial strain of lactic acid bacteria, Lactobacillus sp. B001 per se, having a high capacity of producing mannitol, a method of producing mannitol using this strain.

Being in the form of a white crystal or powder, mannitol has a degree of sweetness 30-40% that of sucrose, cooling sweetness, and very low hygroscopicity. Mannitol is generally used as a diluent or filler of pharmaceuticals and preparations. For example, mannitol is used for intermediates of hypotensive drugs and in producing electrolytic condensers, as well as used for surface tack eliminators for chewing gums and candies, being an industrially useful substance.

Examples of methods of producing mannitol generally include a method of collecting mannitol from natural substances e.g., mannan, an ingredient of seaweeds, by extracting it with hot ethanol [as described in ENCYCLOPAEDIA CHIMICA, Vol.8, pp.924-925(1968)]; and an industrial method of reducing fructose or sucrose catalytically [as described in Shokuhin Tenkabutu Binran, p.387 (1968)]. A variety of methods of producing mannitol, such as a chemical synthesis, a fermentation method and an enzyme method are described in Japanese Laid Open Patent Application (Kokai) No. 239995/87.

Recently, mannitol has been produced by reducing sucrose under high pressure, separating sorbitol prepared in this process as a by-product using an ion exchange resin, and crystallizing the obtained material. However, this method has a drawback in which a unit cost is relatively high.

A microorganism which produces mannitol is known previously. For example, a method of preparing mannitol from a fermented medium in which a microorganism was cultured is described in ENCYCLOPAEDIA CHIMICA, Vol.8, pp. 924-925 (1968). This method is reported by W. H. Peterson in 1920. In the fermentation method, fructose is reduced by the reductive pyridine nucleotide, which derived from the hexose monophosphate shunt by $\alpha$ -hetero lactic acid bacteria, to form mannitol. In general, it is thought that the reaction is proceeded according to the following process.

2 fructose + 1 glucose or fructose $\rightarrow$ 2 mannitol + lactic acid + acetic acid + carbon dioxide + water.

Today microorganisms producing mannitol are known to be lactic acid bacteria, yeasts and fungi. Examples of lactic acid bacteria include Lactobacillus brevis, Leuconostoc mesenteroides, Lactobacillus fermentum, Leuconostoc dextrancam and the like. Examples of yeasts include Saccaromyces sake, genus Torulopsis, Cryptococcus neoformans, Candida lipolitica and the like. Examples of fungi include Ascomycetes such as genus Aspergillus and genus Penicillium, and the like.

These strains of the bacteria produce mannitol from sugars such as fructose and glucose as a substrate by the mannitol fermentation, and among them some strains have a yield per sugar of at least 50%. However, there was generally a problem that when an initial sugar concentration was at least 10% to increase the productivity, the reaction was discontinued to leave sugar. Furthermore, there was a problem that it is difficult to obtain mannitol with a high yield and productivity because the period of culture required 3 to 5 days for bacteria, especially no less then 12 days for fungi.

Thus it has been desired that a microorganism should be found which have high productivity of mannitol, i.e., a capacity of producing mannitol in a short period even under high concentrations of substrates.

SUMMARY OF THE INVENTION

The present invention is accomplished to overcome the above mentioned problems. The object of the present invention is to provide a lactic acid bacterium which has high productivity of mannitol, i.e., a capacity of producing mannitol in a short period even under high concentrations of substrates, and to provide a method of producing mannitol using the bacterium.

Thus the present invention provides Lactobacillus sp. B001 (FERM BP-3158) as a lactic acid bacterium which has high productivity of mannitol, i.e., a capacity of producing mannitol in a short period even under high concentrations of substrates.

The present invention also provides a method of producing mannitol using Lactobacillus sp. B001 (FERM BP-3158).

Lactobacillus sp. B001 according to the present invention is a bacterial strain which has a capacity of producing mannitol with a extremely short period and high yield. Mannitol can be produced by the method using this bacterial strain with a higher yield and more inexpensively than by the other methods of producing mannitol according to the fermentation procedure and the catalytic reduction method.

EP 0 486 024 B1

DETAILED DESCRIPTION OF THE INVENTION

The first embodiment of the present invention is a bacterial strain belonging to genus Lactobacillus having a high capacity of producing mannitol, which also produce lactic acid and acetic acid. This bacterial strain is Lactobacillus sp. B001 deposited as FERM BP-3158 in Fermentation RESEARCH Institute Agency of Industrial Science and Technology in Japan.

The second embodiment of the present invention is a method of producing mannitol using Lactobacillus sp. B001. The present invention is illustrated further in detail in the following.

The present inventors searched for a microorganism having a high capacity of producing mannitol to find Lactobacillus sp. B001 (FERM BP-3158) which has high productivity of mannitol, i.e., a capacity of producing mannitol in a short period even under high concentrations of substrates. This new bacterial strain, Lactobacillus sp. B001, is a fermenting hetero lactic acid bacterium which produces lactic acid, acetic acid and mannitol.

Furthermore, the present inventors found that when Lactobacillus sp. B001 was cultured in a medium containing sugars and yeast extracts at 37°C, mannitol was produced with a yield per sugar of at least 60% within 24 hours.

The bacteriological properties of Lactobacillus sp. B001 according to the present invention are listed as follows.

(A) Morphology of the bacterium

(1) Size of the bacterium: 0.7-1.0μm × 1.2-4.0μm
(2) Form of the bacterium: rod
(3) Mobility: none
(4) Sporulation: none
(5) Gram stain: positive

(B) Growth conditions in the medium (the morphology of the bacterium when it is smeared in a MRS agar medium and cultured at 37 °C for 48 hours)

(1) Form: circle
(2) Size: 0.5-1.5 mm
(3) Elevation: in the form of fried eggs
(4) Color tone: translucent (milky)

(C) physiological properties

(1) Growth temperature: optimal temperature, 35-40 °C growth range, 15-45°C
(2) Growth pH: optimal range, 5.0-6.5 growth range, 4.0-7.0
(3) Behavior to oxygen: facultative anaerobic
(4) Nitrate cannot be reduced
(5) Generation of pigment: none
(6) Generation of indol: none
(7) Generation of hydrogen sulfide: none
(8) Degradation of starch: negative
(9) Degradation of casein: negative
(10) Degradation of gelatin: negative
(11) Degradation of arginine: negative
(12) Degradation of DNA: positive (weak)
(13) Catalase: negative
(14) Oxydase: negative
(15) Litmus milk: unchanged
(16) Methylene Blue: does not grow
(17) Resistance to sodium chloride: does not grow in 6.5% NaCl
(18) Fermentation: heterolactic
(19) Acid and gas generation of sugars

3

|  | Acid generation | Gas generation |
|---|---|---|
| 1. Arabinose | + | − |
| 2. Xylose | + | − |
| 3. Rhamnose | − | − |
| 4. Ribose | + | − |
| 5. Glucose | + | + |
| 6. Mannose | − | − |
| 7. Fructose | + | − |
| 8. Galactose | + | − |
| 9. Sucrose | + | − |
| 10. Maltose | + | − |
| 11. Cellobiose | − | − |
| 12. Lactose | − | − |
| 13. Trehalose | − | − |
| 14. Melibiose | + | − |
| 15. Raffinose | + | − |
| 16. Melezitose | − | − |
| 17. Mannitol | − | − |
| 18. Sorbitol | − | − |
| 19. Escline | − | − |
| 20. Salicin | − | − |
| 21. Amygdalin | − | − |
| 22. Gluconic acid | + | + |

When these properties were examined on the basis of Bergey's manual of determinative bacteriology 8th ed. (1974) and Bergey's manual of systematic bacteriology, Vol.2 (1974), the strain of the present invention belongs to genus Lactobacillus of hetero lactic acid bacteria type, but does not belong to genus Leuconostoc or genus Etysipeothrix, because this strain is a Gram-positive, non-spore, facultative anaerobic, and catalase-negative rod; produces gas from glucose fermentation; and sometimes is in the form of coccobacillus and usually of bacillus, but is not in the form of filament. The strain of the present invention metabolizes melibiose, but not melezitose and cellobiose. There-

4

fore, the strain of the present invention is thought to be a bacterium similar to Lactobacillus brevis.

However, the strain of the present invention metabolizes fructose easily, but not mannitol. Although Lactobacillus brevis is said not to grow at 45 °C, the strain of the present invention grows well or rapidly at 45 °C and poorly at 15 °C, and produces mannitol very rapidly; therefore this strain is different from Lactobacillus brevis, so it is classified into Lactobacillus sp. B001.

The strain of the present invention grows actively even at 45 °C and produces lactic acid, acetic acid and mannitol in a broad temperature range of 30°C to 45°C at a very short period. This strain can produce particularly mannitol with a yield per sugar of at least 60%.

The strain of the present invention grows rapidly in a broad pH range of 4 to 7, and produces mannitol in a pH range of 5 to 7 with a extremely high yield.

Furthermore, the strain of the present invention does not discontinue fermentation even at an initial sugar concentration of at least 20%, and produces mannitol at a short period depending on the initial sugar concentration.

In the method of producing mannitol using Lactobacillus sp. B001 (FERM BP-3158) according to the present invention, mannitol can be produced with a high yield per sugar of at least 60%, when Lactobacillus sp. B001 (FERM BP-3158) is cultured in the medium containing sugars being an admixture of 1 part glucose and 2 part fructose, and yeast extracts in a optimal pH range of 4 to 7, preferably 5 to 7, for 24 hours.

Example 1

Five milliliters of the medium containing ingredients shown in (1) of Table 1 were introduced to a test tube, and was heat-sterilized at 121°C for 15 minutes. One loop of the conserved Lactobacillus sp. B001 (FERM BP-3158) was inoculated to the medium, which was cultured at 37°C for 15 hours to obtain a primary culture.

Fifty milliliters of the medium were introduced to 100 ml-conical flask, and heat-sterilized at 121°C for 5 minutes. The primary culture (0.5 ml) was added to the resulting medium, and cultured at 37 °C for 8 hours to obtain a preliminary culture.

Two liters of the medium containing ingredients shown in (2) of Table 1 were introduced to a 5-liter minijar fermentor, and was heat-sterilized at 121°C for 15 minutes. Twenty milliliters of the preliminary medium were then added to the medium, and the resulting medium was cultured at 37 °C under slightly stirring. While culturing, the pH of the medium was adjusted to 6.0 with 5 N NaOH.

Table 1

| ingredient | medium (1) | medium (2) |
|---|---|---|
| Glucose | - | 66.7 g/l |
| Fructose | 20 g/l | 133.3 g/l |
| Polypepton | 10 g/l | 10 g/l |
| Yeast extract | 5 g/l | 5 g/l |
| Meat extract | 3 g/l | 3 g/l |
| Tween 80 | 1 g/l | 1 g/l |
| $KH_2PO_4$ | 1 g/l | 1 g/l |
| $K_2HPO_4$ | 1 g/l | 1 g/l |
| $MgSO_4 \cdot 7H_2O$ | 0.58 g/l | 0.58 g/l |
| $MnSO_4 \cdot 2H_2O$ | 0.12 g/l | 0.12 g/l |
| $FeSO_4 \cdot 7H_2O$ | 0.034 g/l | 0.034 g/l |

After Lactobacillus sp. B001 (FERM BP-3158) was cultured, glucose, fructose and the obtained mannitol in the medium were assayed by liquid chromatography.

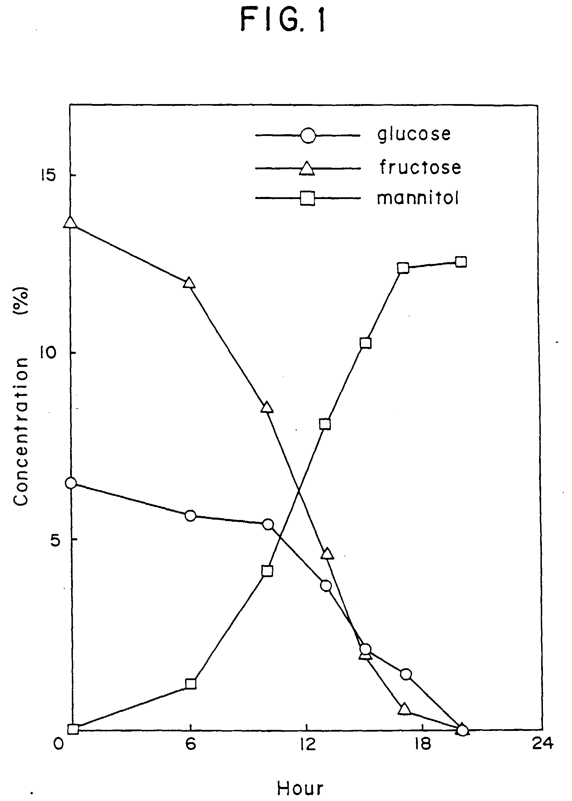

The results of the culture are shown in Fig.1.

Fig.1 is a graph which indicates concentrations of glucose, fructose and the obtained mannitol relative to time. As shown in Fig.1, Lactobacillus sp. B001 (FERM BP-3158) does not discontinue fermentation even under the condition of the initial sugar concentration of 20%, and produced 12.8% mannitol in 20 hours. An extremely high yield was attained in the mannitol fermentation, with a yield per sugar of as much as 64%.

Example 2

Since the medium used in Example 1 is expensive, it is unsuitable for the industrial scale, though there is no problem in term of the culturing time and yield.

In Example 2, the culture was performed with the medium suitable for industrial scale using corn steep liquor as a source of nitrogen, mineral and the like, and using isomerized sugar as a source of carbon.

Two liters of the medium shown in Table 2 were introduced to a 5-liter minijar fermentor, and was heat-sterilized at 100°C for 30 minutes. Twenty milliliters of the preliminary medium cultured by the same method as in Example 1 were inoculated to the medium, and the obtained medium was cultured at 37°C under slightly stirring. While culturing, the pH of the medium was adjusted to 6.2 with 5 N NaOH. The assay was performed by liquid chromatography as in Example 1.

Table 2

| ingredient | |
|---|---|
| isomerized sugar<br>  glucose:fructose = 1:2<br>  solid matter = 76% by weight | 264 g/l |
| corn steep liquor<br>  solid matter = 48% by weight | 50 g/l |

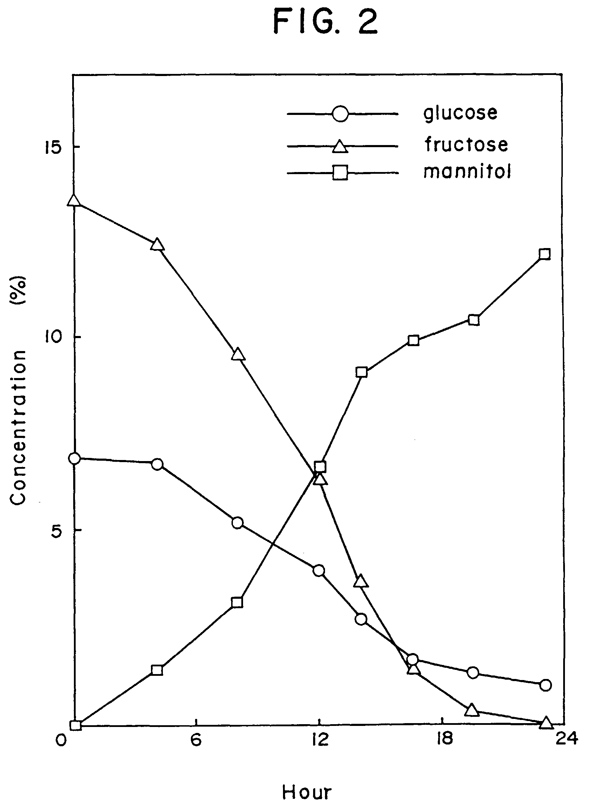

The results of the culture are shown in Fig.2. As shown in Fig.2, Lactobacillus sp. B001 (FERM BP-3158) produced 12.3% mannitol in 23 hours with a yield per sugar of 61%. Since mannitol can be produced even in an inexpensive medium with a very short time and high yield, mannitol may be provided inexpensively in an industrial scale.

Example 3

As shown in Example 1 and Example 2, Lactobacillus sp. B001 (FERM BP-3158) of the present invention is an excellent mannitol-producing strain. In a comparative example, when Lactobacillus brevis (IFO 3960), an another well-known mannitol-producing strain, which was described in Japanese Laid Open Patent (Kokai) No.239995/87, was cultured in a medium containing 5% glucose and 10% fructose (supplemented by betaine) at pH 5.5 at 30°C for 40 hours, about 9.5% mannitol was obtained with a yield per sugar of 65%.

Furthermore, when Leuconostoc mesenteroides (ATCC 8287, IFO 3345) was cultured in a medium containing 10% glucose and 20% fructose for 72 hours, about 19% mannitol was be obtained with a yield per sugar of 63%. The culturing time could be shortened to 60 hours by supplementing 0.1% betaine. The cultures of Lactobacillus sp. B001 (FERM BP-3158) of the present invention and the above mentioned Lactobacillus brevis (IFO 3960) were then compared in the following Example.

The ingredients of the medium used in the present Example are shown in Table 3.

Table 3

| ingredient | |
|---|---|
| Glucose | 50 g/l |
| Fructose | 100 g/l |
| Polypepton | 10 g/l |
| Yeast extract | 5 g/l |
| Meat extract | 3 g/l |
| Tween 80 | 1 g/l |
| $KH_2PO_4$ | 1 g/l |
| $K_2HPO_4$ | 1 g/l |
| $MgSO_4 \cdot 7H_2O$ | 0.58 g/l |
| $MnSO_4 \cdot 2H_2O$ | 0.12 g/l |
| $FeSO_4 \cdot 7H_2O$ | 0.034 g/l |

Two hundred milliliters of the medium shown in Table 3 were introduced to a 300 milliliter-conical flask, and were heat-sterilized at 121°C for 15 minutes. Two milliliters of the preliminary medium cultured by the same methods as in Example 1 and Example 2 were added to the medium, and the resulting medium was cultured at 37 °C under stirring. While culturing, the pH of the medium was adjusted to 6.0 with 5 N NaOH. Glucose, fructose and the obtained mannitol in the medium were assayed by liquid chromatography. The obtained results are shown in Table 4.

Table 4

| Comparative cultures of Lactobacillus sp. B001 (FERM BP-3158) and Lactobacillus brevis (IFO 3960) | | | |
|---|---|---|---|
| Lactobacillus sp. B001 (FERM BP-3158) | | | |
| hour | glucose (%) | fructose (%) | mannitol(%) |
| 0 | 5.1 | 10.0 | 0 |
| 20 | 0 | 0 | 9.2 |
| | | | |
| Lactobacillus brevis (IFO 3960) | | | |
| hour | glucose (%) | fructose (%) | mannitol(%) |
| 0 | 5.0 | 10.0 | 0 |
| 20 | - | - | - |
| 24 | 4.3 | 6.6 | 2.2 |
| 40 | 3.9 | 3.7 | 5.2 |
| 48 | 3.0 | 2.1 | 5.8 |
| 68 | 2.3 | 0.9 | 6.0 |
| 120 | 0.6 | 0.6 | 6.3 |

Lactobacillus sp. B001 (FERM BP-3158) of the present invention does not discontinue fermentation even under the condition of the initial sugar concentration of 15%, and produced 9.2% mannitol in no more than 20 hours with a yield per sugar of 61%. On the other hand, Lactobacillus brevis (IFO 3960) needed 120 hours to produce 7.0% mannitol and had a yield per sugar of 46%, leaving sugar.

It is clearly shown that the bacterium of the present invention had a high capacity of producing mannitol compared with the results of culturing Lactobacillus brevis (IFO 3960) which was described in Japanese Laid Open Patent (Kokai) No. 239995/87.


**Claims**

1. Lactobacillus sp. B001 (FERM BP-3158) belonging to the genus Lactobacillus, deposited in Fermentation Research Institute Agency of Industrial Science and Technology.

2. A method of producing mannitol, wherein Lactobacillus sp. B001 (FERM BP-3158) of Claim 1 is cultured in a medium containing sugars being an admixture of 2 parts fructose and 1 part glucose.


**Patentansprüche**

1. Lactobazillus sp.B001 (FERM BP-3158) aus der Gattung Lactobazillus, hinterlegt bei der Fermentation Research Institute Agency of Industrial Science and Technology (Fermentationsforschungsinstitutsstelle für Technische Wissenschaft und Technologie).

2. Verfahren zur Herstellung von Mannitol, wobei der Lactobazillus sp.B001 (FERM BP-3158) nach Anspruch 1 in einem Medium gezüchtet wird, welches Zucker enthält, die ein Gemisch aus 2 Teilen Fructose und 1 Teil Glucose sind.

**Revendications**

1.  Lactobacillus espèce B001 (FERM BP-3158) appartenant au genre Lactobacillus, déposé à la Fermentation Research Institute Agency of Industrial Science and Technology (Institut de Recherche en Fermentation à vocation industrielle et technique).

2.  Procédé de production de mannitol, dans lequel Lactobacillus espèce B001 (FERM BP-3158) selon la revendication 1, est mis en culture dans un milieu contenant des sucres constitué d'un mélange de deux parties de fructose et d'une partie de glucose.

# FIG. 1

# FIG. 2